(19) Europäisches Patentamt
European Patent Office
Office européen des brevets

(11) EP 4 066 641 A1

(12) EUROPEAN PATENT APPLICATION

(43) Date of publication:
05.10.2022 Bulletin 2022/40

(21) Application number: 22157915.4

(22) Date of filing: 02.05.2018

(51) International Patent Classification (IPC):
*A01N 43/54* (2006.01) *A01N 43/653* (2006.01)
*C07C 229/08* (2006.01) *A01P 3/00* (2006.01)
*A01N 43/40* (2006.01)

(52) Cooperative Patent Classification (CPC):
(C-Sets available)
A01N 43/40 (Cont.)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 02.05.2017 US 201762500186 P

(62) Document number(s) of the earlier application(s) in accordance with Art. 76 EPC:
18794937.5 / 3 618 625

(71) Applicant: Corteva Agriscience LLC
Indianapolis, IN 46268 (US)

(72) Inventors:
- YAO, Chenglin
Indianapolis, 46268 (US)
- MATHIESON, John T.
Indianapolis, 46268 (US)

(74) Representative: f & e patent
Braunsberger Feld 29
51429 Bergisch Gladbach (DE)

Remarks:
This application was filed on 22-02-2022 as a divisional application to the application mentioned under INID code 62.

# (54) SYNERGISTIC MIXTURES FOR FUNGAL CONTROL IN VEGETABLES

(57) A fungicidal composition containing a fungicidally effective amount of the compound of Formula I, (*S*)-1,1-bis(4-fluorophenyl)propan-2-yl (3-acetoxy-4-methoxypicolinoyl)-L-alaninate, and at least one fungicide selected from the group consisting of epoxiconazole, prothioconazole, difenoconazole, azoxystrobin, pyraclostrobin, picoxoystrobin, fluxapyroxad, benzovindiflupyr, penthiopyrad, bixafen, chlorothalonil, and mancozeb provides synergistic control of selected fungi.

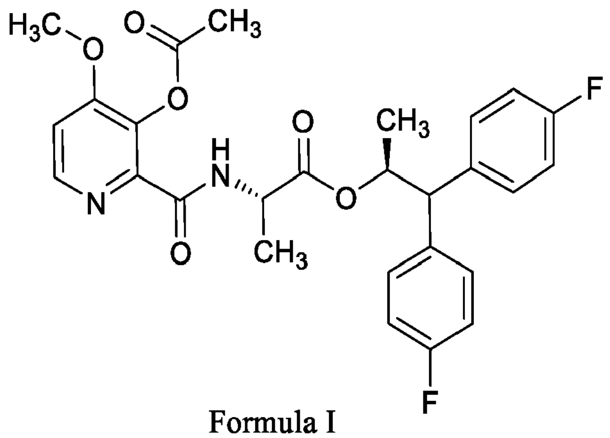

Formula I

EP 4 066 641 A1

(52) Cooperative Patent Classification (CPC): (Cont.)

C-Sets
**A01N 43/40, A01N 37/34, A01N 43/40, A01N 43/54, A01N 43/56, A01N 43/653, A01N 47/14, A01N 47/24**

## Description

CROSS REFERENCE TO RELATED APPLICATIONS

This application claims the benefit of U.S. Provisional Patent Application Serial No. 62/500186 filed May 2, 2017, which is expressly incorporated by reference herein.

FIELD

This disclosure concerns a synergistic fungicidal composition containing (a) the compound of Formula I and (b) at least one fungicide selected from the group consisting of a sterol biosynthesis inhibitor, for example prothioconazole, epoxiconazole, cyproconazole, myclobutanil, metconazole, difenoconazole, tebuconazole, tetraconazole, fenbuconazole, propiconazole, fluquinconazole, flusilazole, and flutriafol; a strobilurin, for example pyraclostrobin, fluoxastrobin, azoxystrobin, trifloxystrobin, picoxystrobin, and kresoxim methyl; a succinate dehydrogenase inhibitor, for example fluxapyroxad, benzovindiflupyr, penthiopyrad, isopyrazam, bixafen, boscalid, penflufen, and fluopyram; a multi-site inhibitor, for example mancozeb and chlorothalonil, or other commercial fungicides to provide control of any plant fungal pathogen.

BACKGROUND AND SUMMARY

Fungicides are compounds, of natural or synthetic origin, which act to protect plants against damage caused by fungi. Current methods of agriculture rely heavily on the use of fungicides. In fact, some crops cannot be grown usefully without the use of fungicides. Using fungicides allows a grower to increase the yield and the quality of the crop, and consequently, increase the value of the crop. In most situations, the increase in value of the crop is worth at least three times the cost of the use of the fungicide.

However, no one fungicide is useful in all situations and repeated usage of a single fungicide frequently leads to the development of resistance to that and related fungicides. Consequently, research is being conducted to produce fungicides and combinations of fungicides that are safer, that have better performance, that require lower dosages, that are easier to use, and that cost less.

Synergism occurs when the activity of two or more compounds exceeds the activities of the compounds when used alone.

It is an object of this disclosure to provide synergistic compositions comprising fungicidal compounds. It is a further object of this disclosure to provide processes that use these synergistic compositions. The synergistic compositions are capable of preventing or curing, or both, diseases caused by fungi of the classes *Ascomycetes* and *Basidiomycetes*. In addition, the synergistic compositions have improved efficacy against the *Ascomycete* and *Basidiomycete* pathogens, including tomato early blight. In accordance with this disclosure, synergistic compositions are provided along with methods for their use.

DETAILED DESCRIPTION

The present disclosure concerns a synergistic fungicidal mixture comprising a fungicidally effective amount of (a) the compound of Formula I and (b) at least one fungicide selected from the compounds of the following groups A.1, B.1 and C.1:

A.1 Sterol biosynthesis inhibitors (SBI fungicides) selected from the following groups a), b) and c):

a) C14 demethylase inhibitors (DMI fungicides), for example prothioconazole, epoxiconazole, cyproconazole, myclobutanil, metconazole, difenoconazole, tebuconazole, tetraconazole, fenbuconazole, propiconazole, fluquinconazole, flusilazole, flutriafol and prochloraz;
b) Delta 14-reductase inhibitors, for example, fenpropimorph and aldimorph;
c) Inhibitors of 3-keto reductase such as fenhexamid;

B.1 Respiration inhibitors selected from the following groups a) and b):

a) inhibitors of complex II (SDHI fungicides, e.g. carboxamides), for example fluxapyroxad, benzovindiflupyr, penthiopyrad, isopyrazam, bixafen, boscalid, penflufen, and fluopyram;
b) inhibitors of complex III at the $Q_o$ site (e.g. strobilurins), for example pyraclostrobin, fluoxastrobin, azoxystrobin, trifloxystrobin, picoxystrobin, and kresoxim methyl;

C.1 Inhibitors with multi-site action selected from the following groups a) and b):

a) thio- and dithiocarbamates, such as mancozeb;
b) organochlorine compounds (e.g. phthalimides, sulfamides, chloronitriles) such as chlorothalonil;

or other commercial fungicides to provide control of any plant fungal pathogen.

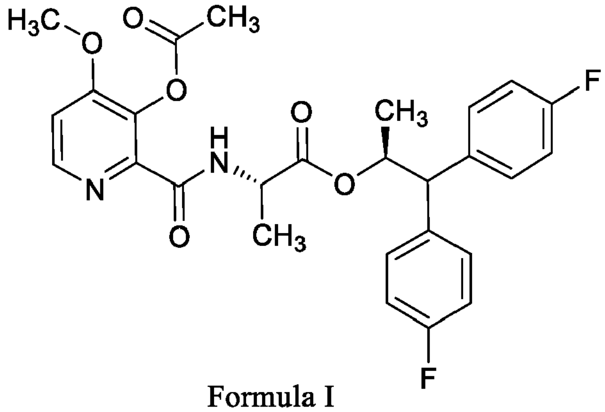

Formula I

As used herein, the compound of Formula I is (*S*)-1,1-bis(4-fluorophenyl)propan-2-yl (3-acetoxy-4-methoxypicolinoyl)-*L*-alaninate. The compound of Formula I provides control of a variety of pathogens in economically important crops including, but not limited to, the causal agent of tomato early blight, *Alternaria solani* (ALTESO).
As used herein, epoxiconazole is the common name for (2*RS*,3*SR*)-1-[3-(2-chlorophenyl)-2,3-epoxy-2-(4-fluorophenyl)propyl]-1*H*-1,2,4-triazole and possesses the following structure:

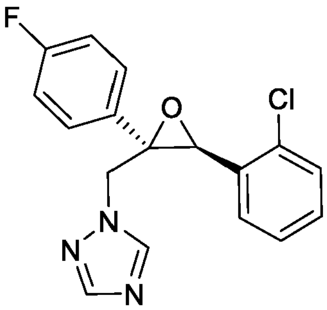

Its fungicidal activity is described in The Pesticide Manual, Fifteenth Edition, 2009. Epoxiconazole provides broad spectrum control, with preventive and curative action, of diseases caused by Ascomycetes, Basidiomycetes and Deuteromycetes in bananas, cereals, coffee, rice and sugar beet.
As used herein, prothioconazole is the common name for 2-[(2*RS*)-2-(1-chlorocyclopropyl)-3-(2-chlorophenyl)-2-hydroxypropyl]-2*H*-1,2,4-triazole-3(4*H*)-thione and possesses the following structure:

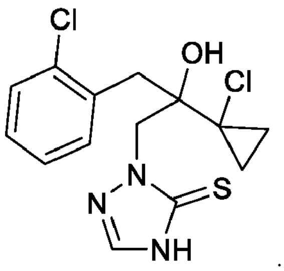

Its fungicidal activity is described in The Pesticide Manual, Fifteenth Edition, 2009. Prothioconazole provides control of diseases such as eyespot (*Pseudocercosporella herpotrichoides*), Fusarium ear blight (*Fusarium* spp., *Micro-*

*dochium nivale*), leaf blotch diseases (*Zymoseptoria tritici*, *Parastagonospora nodorum*, *Pyrenophora* spp., *Rhynchosporium secalis*, etc.), rust (*Puccinia* spp.) and powdery mildew (*Blumeria graminis*), by foliar application, in wheat, barley and other crops.

As used herein, difenoconazole is the common name for 3-chloro-4-[(2*RS*, *4RS, 2RS,* 4*RS*)-4-methyl-2-(1*H*-1,2,4-triazol-1-ylmethyl)-1,3-dioxolan-2-yl]phenyl 4-chlorophenyl ether and possesses the following structure:

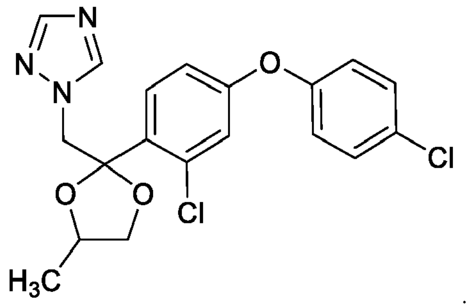

Its fungicidal activity is described in BCPC Online Pesticide Manual - Latest Version. Difenoconazole provides broad spectrum control, with preventive and curative action, of diseases caused by Ascomycetes, Basidiomycetes and Deuteromycetes in grapes, pome fruit, stone fruit, potatoes, sugar beet, oilseed rape, bananas, cereals, rice, soybeans, ornamentals and various vegetable crops.

As used herein, azoxystrobin is the common name for (*E*)-2-{2-[6-(2-cyanophenoxy)pyrimidin-4-yloxy]phenyl}-3-methoxyacrylate and possesses the following structure:

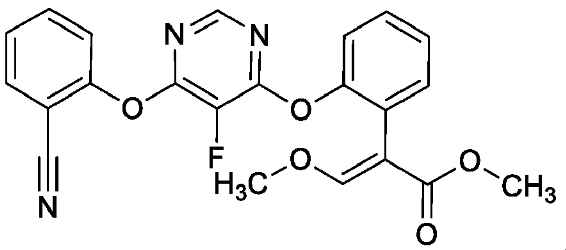

Its fungicidal activity is exemplified in The e-Pesticide Manual, Version 5.2, 2011. Exemplary uses of azoxystrobin include, but are not limited to, control of the following pathogens: *Erysiphe graminis*, *Puccinia* spp., *Parastagonospora nodorum*, *Zymoseptoria tritici* and *Pyrenophora teres* on temperate cereals; *Pyricularia oryzae* and *Rhizoctonia solani* on rice; *Plasmopara viticola* and *Uncinula necator* on vines; *Sphaerotheca fuliginea* and *Pseudoperonospora cubensis* on cucurbitaceae; *Phytophthora infestans* and *Alternaria solani* on potato and tomato; *Mycosphaerella arachidis*, *Rhizoctonia solani* and *Sclerotium rolfsii* on peanut; *Monilinia* spp. and *Cladosporium carpophilum* on peach; *Pythium* spp. and *Rhizoctonia solani* on turf; *Mycosphaerella* spp. on banana; *Cladosporium caryigenum* on pecan; *Elsinoe fawcettii*, *Colletotrichum* spp. and *Guignardia citricarpa* on citrus; *Colletotrichum* spp. and *Hemileia vastatrix* on coffee.

As used herein, pyraclostrobin is the common name for methyl *N*-[2-[[[1-(4-chlorophenyl)-1*H*-pyrazol-3-yl]oxy]methyl]phenyl]-*N*-methoxycarbamate and possesses the following structure:

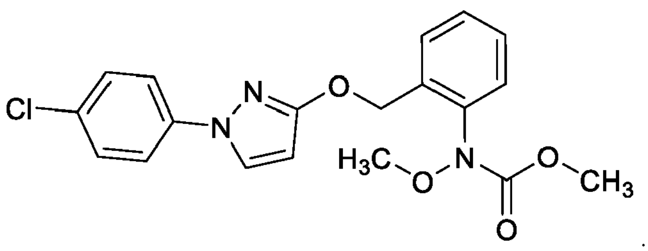

Its fungicidal activity is described in BCPC Online Pesticide Manual - Latest Version. Exemplary uses of pyraclostrobin include, but are not limited to, broad spectrum disease control of major plant pathogens, including *Zymoseptoria tritici*, *Puccinia* spp., *Drechslera tritici-repentis*, *Pyrenophora teres, Rhynchosporium secalis* and *Septoria nodorum* in

cereals; *Mycosphaerella* spp. in peanuts; *Septoria glycines*, *Cercospora kikuchii* and *Phakopsorapachyrhizi* in soybeans; *Plasmopara viticola* and *Erysiphe necator* in grapes; *Phytophthora infestans* and *Alternaria solani* in potatoes and tomatoes; *Sphaerotheca fuliginea* and *Pseudoperonospora cubensis* in cucumber; *Mycosphaerella fijiensis* in bananas; *Elsinoe fawcettii* and *Guignardia citricarpa* in citrus and *Rhizoctonia solani* and *Pythium aphanidermatum* in turf.

As used herein, picoxystrobin is the common name for methyl (*E*)-3-methoxy-2-[2-(6-trifluoromethyl-2-pyridyloxymethyl)phenyl]acrylate and possesses the following structure:

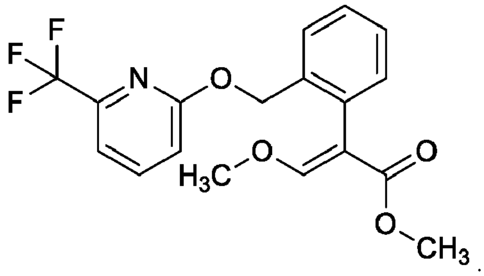

Its fungicidal activity is described in The e-Pesticide Manual, Version 5.2, 2011. Exemplary uses of picoxystrobin include, but are not limited to, broad-spectrum disease control in cereals, including *Mycosphaerella graminicola*, *Phaeosphaeria nodorum*, *Puccinia recondita* (brown rust), *Helminthosporium tritici-repentis* (tan spot) and *Blumeria graminis* f.sp. *tritici* (strobilurin-sensitive powdery mildew) in wheat; *Helminthosporium teres* (net blotch), *Rhynchosporium secalis*, *Puccinia hordei* (brown rust) and *Erysiphe graminis* f.sp. *hordei* (strobilurin-sensitive powdery mildew) in barley; *Puccinia coronata* and *Helminthosporium avenae* in oats; and *Puccinia recondita* and *Rhynchosporium secalis* in rye.

As used herein, fluxapyroxad is the common name for 3-(difluoromethyl)-1-methyl-*N*-(3',4',5'-trifluorobiphenyl-2-yl)pyrazole-4-carboxamide and possesses the following structure:

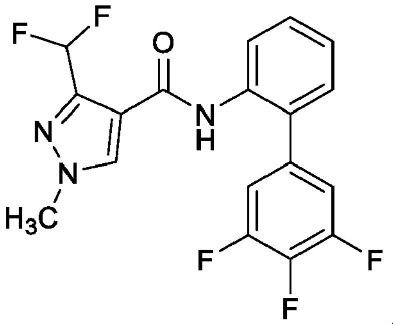

Its fungicidal activity is exemplified in Agrow Intelligence (https://www.agra-net.net/agra/agrow/databases/agrow-intelligence/). Exemplary uses of fluxapyroxad include, but are not limited to, the control of plant pathogens, such as *Helminthosporium teres* (net blotch), *Rhynchosporium secalis* (leaf scald), *Puccinia hordei* (brown rust), and *Erysiphe graminis* f.sp. *hordei* (powdery mildew) in a range of crops, such as barley, maize, and soybeans.

As used herein, benzovindiflupyr is the common name for *N*-[(1*RS*,4*SR*)-9-(dichloromethylene)-1,2,3,4-tetrahydro-1,4-methanonaphthalen-5-yl]-3-(difluoromethyl)-1-methylpyrazole-4-carboxamide and possesses the following structure:

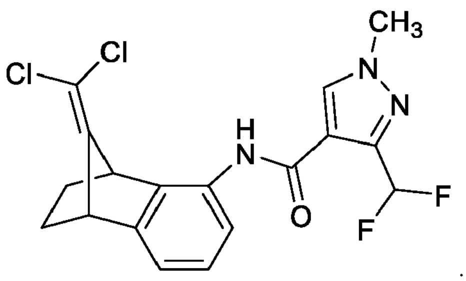

Its fungicidal activity is exemplified in Agrow Intelligence (https://www.agra-net.net/agra/agrow/databases/agrow-intelligence/). Exemplary uses of benzovindiflupyr include, but are not limited to, controlling a variety of pathogens such as *Botrytis* spp., *Erysiphe* spp., *Rhizoctonia* spp., *Septoria* spp., *Phytophthora* spp., *Pythium* spp., *Phakopsora pachyrhizi*, and *Puccinia recondita*, in a range of crops including vines, cereals, soybeans, cotton, and fruit and vegetable crops.

As used herein, penthiopyrad is the common name for *N*-[2-(1,3-dimethylbutyl)-3-thienyl]-1-methyl-3-(trifluoromethyl)-1*H*-pyrazole-4-carboxamide and possesses the following structure:

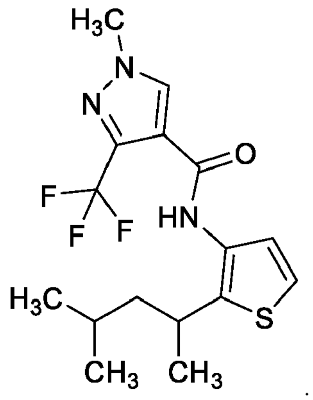

Its fungicidal activity is described in The Pesticide Manual, Fourteenth Edition, 2006. Penthiopyrad provides control of rust and Rhizoctonia diseases, as well as grey mold, powdery mildew and apple scab.

As used herein, bixafen is the common name for *N*-(3,4-dichloro-5-fluoro[1,1'-biphenyl]-2-yl)-3-(difluoromethyl)-1-methyl-1*H*-pyrazole-4-carboxamide and possesses the following structure:

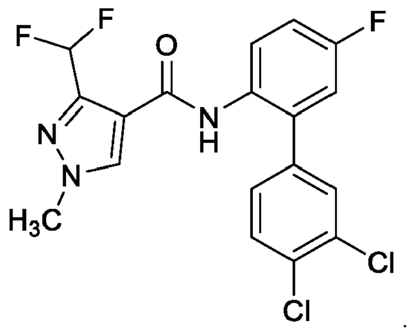

Its fungicidal activity is described in BCPC Online Pesticide Manual - Latest Version. Exemplary uses of bixafen include, but are not limited to, broad-spectrum disease control in cereals, including *Zymoseptoria tritici*, *Puccinia triticina, Puccinia striiformis*, *Oculimacula* spp. and *Pyrenophora tritici-repentis* in wheat and against *Pyrenophora teres*, *Ramularia collo-cygni, Rhynchosporium secalis* and *Puccinia hordei* in barley.

As used herein, chlorothalonil is the common name for tetrachloroisophthal-onitrile and possesses the following structure:

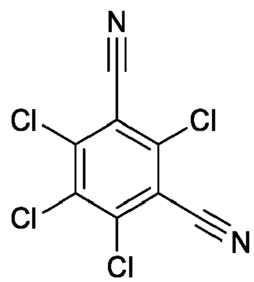

Its fungicidal activity is described in The Pesticide Manual, Fifteenth Edition, 2009. Chlorothalonil provides control of many fungal diseases in a wide range of crops, including pome fruit, stone fruit, almonds, citrus fruit, bush and cane fruit, cranberries, strawberries, pawpaws, bananas, mangoes, coconut palms, oil palms, rubber, pepper, vines, hops, vegetables, cucurbits, tobacco, coffee, tea, rice, soya beans, peanuts, potatoes, sugar beet, cotton, maize, ornamentals, mushrooms, and turf.

As used herein, mancozeb is the common name for [[2-[(dithiocarboxy)amino]ethyl]carbamodithioato(2-)-κ*S*,κ*S'*]manganese mixture with [[2-[(dithiocarboxy)amino]ethyl]carbamodithioato(2-)-κ*S*,κ*S'*]zinc and possesses the following structure:

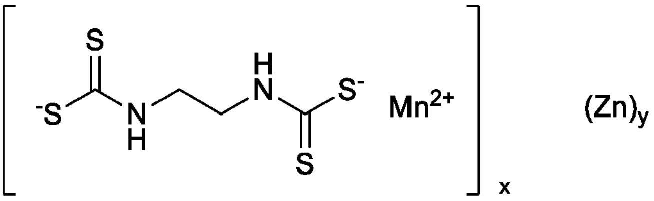

**x:y =** 1 : **0.091**

Its fungicidal activity is described in The Pesticide Manual, Fifteenth Edition, 2009. Mancozeb provides control of a wide range of fungal pathogens on a variety of fruits, vegetables and field crops.

In the compositions described herein, the concentration ratio of the mixture of the compound of Formula I to other fungicides at which the fungicidal effect is synergistic against tomato early blight caused by *Alternaria solani* (ALTESO) in protectant applications lies within the range from about 50:1 to about 1:20.

In the compositions described herein, the concentration ratio of the mixture of the compound of Formula I to a sterol biosynthesis inhibitor at which the fungicidal effect is synergistic against ALTESO in protectant applications lies within the range from about 10:1 to about 1:1.6. In one embodiment, the concentration ratio of the mixture of the compound of Formula I to epoxiconazole at which the fungicidal effect is synergistic against ALTESO in protectant applications is from about 10:1 to about 1:1.6. In another embodiment, the concentration ratio of the mixture of the compound of Formula I to prothioconazole at which the fungicidal effect is synergistic against ALTESO in protectant applications is from about 10:1 to about 1.25:1. In yet another embodiment, the concentration ratio of the mixture of the compound of Formula I to difenoconazole at which the fungicidal effect is synergistic against ALTESO in protectant applications is from about 5:1 to about 1:1.6.

In the compositions described herein, the concentration ratio of the mixture of the compound of Formula I to a strobilurin fungicide at which the fungicidal effect is synergistic against ALTESO in protectant applications lies within the range from about 50:1 to about 1:1.6. In one embodiment, the concentration ratio of the mixture of the compound of Formula I to azoxystrobin at which the fungicidal effect is synergistic against ALTESO in protectant applications lies within the range from about 50:1 to about 3:1. In another embodiment, the concentration ratio of the mixture of the compound of Formula I to pyraclostrobin at which the fungicidal effect is synergistic against ALTESO in protectant applications lies within the range from about 50:1 to about 3:1. In yet another embodiment, the concentration ratio of the mixture of the compound of Formula I to picoxystrobin at which the fungicidal effect is synergistic against ALTESO in protectant applications lies within the range from about 10:1 to about 1:1.6.

In the compositions described herein, the concentration ratio of the mixture of the compound of Formula I to a succinate dehydrogenase inhibitor (SDHI) fungicide at which the fungicidal effect is synergistic against ALTESO in protectant applications lies within the range from about 50:1 to about 1:1.6. In one embodiment, the concentration ratio of the mixture of the compound of Formula I to fluxapyroxad at which the fungicidal effect is synergistic against ALTESO in protectant applications lies within the range from about 50:1 to about 3:1. In another embodiment, the concentration ratio of the mixture of the compound of Formula I to benzovindiflupyr at which the fungicidal effect is synergistic against ALTESO in protectant applications is from about 10:1 to about 1:1.6. In other embodiments, the concentration ratio of the mixture of the compound of Formula I to penthiopyrad at which the fungicidal effect is synergistic against ALTESO in protectant applications is from about 10:1 to about 1.3:1. In yet another embodiment, the concentration ratio of the mixture of the compound of Formula I to bixafen at which the fungicidal effect is synergistic against ALTESO in protectant applications is from about 10:1 to about 1:1.6.

In the compositions described herein, the concentration ratio of the mixture of the compound of Formula I to a multi-site inhibitor fungicide at which the fungicidal effect is synergistic against ALTESO in protectant applications lies within the range from about 1:1.3 to about 1:20. In one embodiment, the concentration ratio of the mixture of the compound of Formula I to chlorothalonil at which the fungicidal effect is synergistic against ALTESO in protectant applications lies within the range from about 1:1.3 to about 1:20. In another embodiment, the concentration ratio of the mixture of the compound of Formula I to mancozeb at which the fungicidal effect is synergistic against ALTESO in protectant applications lies within the range from about 1:1.3 to about 1:20.

The rate at which the synergistic composition is applied will depend upon the particular type of fungus to be controlled, the degree of control required and the timing and method of application. In general, the compositions described herein can be applied at an application rate of between about 40 grams per hectare (g/ha) and about 2650 g/ha based

on the total amount of active ingredients in the composition.

The compositions comprising the compound of Formula I and a sterol biosynthesis inhibitor can be applied at an application rate of between about 40 g/ha and about 400 g/ha based on the total amount of active ingredients in the composition. Epoxiconazole is applied at a rate of between about 50 g/ha and about 250 g/ha and the compound of Formula I is applied at a rate between about 10 g/ha and about 150 g/ha. Prothioconazole is applied at a rate of between about 50 g/ha and about 250 g/ha and the compound of Formula I is applied at a rate between about 10 g/ha and about 150 g/ha. Difenoconazole is applied at a rate of between about 30 g/ha and about 125 g/ha and the compound of Formula I is applied at a rate between about 10 g/ha and about 150 g/ha.

The compositions comprising the compound of Formula I and a strobilurin fungicide can be applied at an application rate of between about 40 g/ha and about 350 g/ha based on the total amount of active ingredients in the composition. Azoxystrobin is applied at a rate of between about 30 g/ha and about 200 g/ha and the compound of Formula I is applied at a rate between about 10 g/ha and about 150 g/ha. Pyraclostrobin is applied at a rate of between about 30 g/ha and about 200 g/ha and the compound of Formula I is applied at a rate between about 10 g/ha and about 150 g/ha. Picoxystrobin is applied at a rate of between about 30 g/ha and about 200 g/ha and the compound of Formula I is applied at a rate between about 10 g/ha and about 150 g/ha.

The compositions comprising the compound of Formula I and a carboxamide SDHI fungicide can be applied at an application rate of between about 40 g/ha and about 350 g/ha based on the total amount of active ingredients in the composition. Fluxapyroxad is applied at a rate of between about 45 g/ha and about 200 g/ha and the compound of Formula I is applied at a rate between about 10 g/ha and about 150 g/ha. Benzovindiflupyr is applied at a rate of between about 45 g/ha and about 200 g/ha and the compound of Formula I is applied at a rate between about 10 g/ha and about 150 g/ha. Penthiopyrad is applied at a rate of between about 45 g/ha and about 200 g/ha and the compound of Formula I is applied at a rate between about 10 g/ha and about 150 g/ha. Bixafen is applied at a rate of between about 30 g/ha and about 200 g/ha and the compound of Formula I is applied at a rate between about 10 g/ha and about 150 g/ha.

The compositions comprising the compound of Formula I and a multi-site inhibitor can be applied at an application rate of between about 1010 g/ha and about 2650 g/ha based on the total amount of active ingredients in the composition. Chlorothalonil is applied at a rate of between about 1000 g/ha and about 2500 g/ha and the compound of Formula I is applied at a rate between about 10 g/ha and about 150 g/ha. Mancozeb is applied at a rate of between about 1000 g/ha and about 2500 g/ha and the compound of Formula I is applied at a rate between about 10 g/ha and about 150 g/ha.

The components of the synergistic mixture described herein can be applied either separately or as part of a multipart fungicidal system.

The synergistic mixture of the present disclosure can be applied in conjunction with one or more other fungicides to control a wider variety of undesirable diseases. When used in conjunction with other fungicide(s), the presently claimed compounds may be formulated with the other fungicide(s), tank mixed with the other fungicide(s) or applied sequentially with the other fungicide(s). Such other fungicides may include 2-(thiocyanatomethylthio)-benzothiazole, 2-phenylphenol, 8-hydroxyquinoline sulfate, ametoctradin, amisulbrom, antimycin, *Ampelomyces quisqualis*, azaconazole, *Bacillus subtilis*, *Bacillus subtilis* strain QST713, benalaxyl, benomyl, benthiavalicarb-isopropyl, benzylaminobenzene-sulfonate (BABS) salt, bicarbonates, biphenyl, bismerthiazol, bitertanol, blasticidin-S, borax, Bordeaux mixture, boscalid, bromuconazole, bupirimate, calcium polysulfide, captafol, captan, carbendazim, carboxin, carpropamid, carvone, chlazafenone, chloroneb, chlozolinate, *Coniothyrium minitans*, copper hydroxide, copper octanoate, copper oxychloride, copper sulfate, copper sulfate (tribasic), cuprous oxide, cyazofamid, cyflufenamid, cymoxanil, cyproconazole, cyprodinil, dazomet, debacarb, diammonium ethylenebis-(dithiocarbamate), dichlofluanid, dichlorophen, diclocymet, diclomezine, dichloran, diethofencarb, difenzoquat ion, diflumetorim, dimethomorph, dimoxystrobin, diniconazole, diniconazole-M, dinobuton, dinocap, diphenylamine, dipymetitrone, dithianon, dodemorph, dodemorph acetate, dodine, dodine free base, edifenphos, enestrobin, enestroburin, ethaboxam, ethoxyquin, etridiazole, famoxadone, fenamidone, fenarimol, fenbuconazole, fenfuram, fenhexamid, fenoxanil, fenpiclonil, fenpropidin, fenpropimorph, fenpyrazamine, fentin, fentin acetate, fentin hydroxide, ferbam, ferimzone, fluazinam, fludioxonil, fluindapyr, flumorph, fluopicolide, fluopyram, fluoroimide, fluoxastrobin, fluquinconazole, flusilazole, flusulfamide, flutianil, flutolanil, flutriafol, folpet, formaldehyde, fosetyl, fosetyl-aluminium, fuberidazole, furalaxyl, furametpyr, guazatine, guazatine acetates, GY-81, hexachlorobenzene, hexaconazole, hymexazol, imazalil, imazalil sulfate, imibenconazole, iminoctadine, iminoctadine triacetate, iminoctadine tris(albesilate), iodocarb, ipconazole, ipfenpyrazolone, iprobenfos, iprodione, iprovalicarb, isofetamide, isoprothiolane, isopyrazam, isotianil, kasugamycin, kasugamycin hydrochloride hydrate, kresoxium-methyl, laminarin, mancopper, mandipropamid, maneb, mefenoxam, mepanipyrim, mepronil, meptyl-dinocap, mercuric chloride, mercuric oxide, mercurous chloride, metalaxyl, metalaxyl-M, metam, metam-ammonium, metam-potassium, metam-sodium, metconazole, methasulfocarb, methyl iodide, methyl isothiocyanate, metiram, metominostrobin, metrafenone, mildiomycin, myclobutanil, nabam, nitrothal-isopropyl, nuarimol, octhilinone, ofurace, oleic acid (fatty acids), orysastrobin, oxadixyl, oxathiapiprolin, oxine-copper, oxpoconazole fumarate, oxycarboxin, pefurazoate, penconazole, pencycuron, penflufen, pentachlorophenol, pentachlorophenyl laurate, phenylmercury acetate, phosphonic acid, phthalide, polyoxin B, polyoxins, polyoxorim, potassium bicarbonate, potassium hydroxyquinoline sulfate, probenazole, prochloraz, procymidone, propamocarb,

propamocarb hydrochloride, propiconazole, propineb, proquinazid, pydiflumetofen, pyrametostrobin, pyraoxystrobin, pyraziflumid, pyrazophos, pyribencarb, pyributicarb, pyrifenox, pyrimethanil, pyriofenone, pyroquilon, quinoclamine, quinoxyfen, quintozene, *Reynoutria sachalinensis* extract, sedaxane, silthiofam, simeconazole, sodium 2-phenylphenoxide, sodium bicarbonate, sodium pentachlorophenoxide, spiroxamine, sulfur, SYP-Z048, tar oils, tebuconazole, tebufloquin, tecnazene, tetraconazole, thiabendazole, thifluzamide, thiophanate-methyl, thiram, tiadinil, tolclofos-methyl, tolylfluanid, triadimefon, triadimenol, triazoxide, tricyclazole, tridemorph, trifloxystrobin, triflumizole, triforine, triticonazole, validamycin, valifenalate, valiphenal, vinclozolin, zineb, ziram, zoxamide, *Candida oleophila*, *Fusarium oxysporum, Gliocladium* spp., *Phlebiopsis gigantea*, *Streptomyces griseoviridis, Trichoderma* spp., (*RS*)-*N*-(3,5-dichlorophenyl)-2-(methoxymethyl)-succinimide, 1,2-dichloropropane, 1,3-dichloro-1,1,3,3-tetrafluoroacetone hydrate, 1-chloro-2,4-dinitronaphthalene, 1-chloro-2-nitropropane, 2-(2-heptadecyl-2-imidazolin-1-yl)ethanol, 2,3-dihydro-5-phenyl-1,4-dithi-ine 1,1,4,4-tetraoxide, 2-methoxyethylmercury acetate, 2-methoxyethylmercury chloride, 2-methoxyethylmercury silicate, 3-(4-chlorophenyl)-5-methylrhodanine, 4-(2-nitroprop-1-enyl)phenyl thiocyanateme, aminopyrifen, ampropylfos, anilazine, azithiram, barium polysulfide, Bayer 32394, benodanil, benquinox, bentaluron, benzamacril; benzamacril-isobutyl, benzamorf, binapacryl, bis(methylmercury) sulfate, bis(tributyltin) oxide, buthiobate, cadmium calcium copper zinc chromate sulfate, carbamorph, CECA, chlobenthiazone, chloraniformethan, chlorfenazole, chlorquinox, climbazole, copper bis(3-phenylsalicylate), copper zinc chromate, cufraneb, cupric hydrazinium sulfate, cuprobam, cyclafuramid, cypendazole, cyprofuram, decafentin, dichlobentiazox, dichlone, dichlozoline, diclobutrazol, dimethirimol, dinocton, dinosulfon, dinoterbon, dipyrithione, ditalimfos, dodicin, drazoxolon, EBP, ESBP, etaconazole, etem, ethirim, fenaminosulf, fenapanil, fenitropan, fluindapyr, fluopimomide, fluotrimazole, furcarbanil, furconazole, furconazole-cis, furmecyclox, furophanate, glyodine, griseofulvin, halacrinate, Hercules 3944, hexylthiofos, ICIA0858, inpyrfluxam, ipfentrifluconazole, ipflufenoquin, isoflucypram, isopamphos, isovaledione, mandestrobin, mebenil, mecarbinzid, mefentrifluconazole, metazoxolon, methfuroxam, methylmercury dicyandiamide, metsulfovax, metyltetraprole, milneb, mucochloric anhydride, myclozolin, *N*-3,5-dichlorophenyl-succinimide, *N*-3-nitrophenylitaconimide, natamycin, *N*-ethylmercurio-4-toluenesulfonanilide, nickel bis(dimethyldithiocarbamate), OCH, phenylmercury dimethyldithiocarbamate, phenylmercury nitrate, phosdiphen, prothiocarb; prothiocarb hydrochloride, pydiflumetofen, pyracarbolid, pyrapropoyne, pyridachlometyl, pyridinitril, pyroxychlor, pyroxyfur, quinacetol; quinacetol sulfate, quinazamid, quinconazole, quinofumelin, rabenzazole, salicylanilide, SSF-109, sultropen, tecoram, thiadifluor, thicyofen, thiochlorfenphim, thiophanate, thioquinox, tioxymid, triamiphos, triarimol, triazbutil, trichlamide, urbacid, zarilamid, and any combinations thereof.

The compositions of the present disclosure are preferably applied in the form of a formulation comprising a composition of (a) a compound of Formula I and (b) at least one fungicide selected from the group consisting of epoxiconazole, prothioconazole, difenoconazole, azoxystrobin, pyraclostrobin, picoxystrobin, fluxapyroxad, benzovindiflupyr, picoxystrobin, bixafen, mancozeb, and chlorothalonil, together with a phytologically acceptable carrier.

Concentrated formulations can be dispersed in water, or another liquid, for application, or formulations can be dust-like or granular, which can then be applied without further treatment. The formulations are prepared according to procedures which are conventional in the agricultural chemical art, but which are novel and important because of the presence therein of a synergistic composition.

The formulations that are applied most often are aqueous suspensions or emulsions. Either such water-soluble, water-suspendable, or emulsifiable formulations are solids, usually known as wettable powders, or liquids, usually known as emulsifiable concentrates, aqueous suspensions, or suspension concentrates. The present disclosure contemplates all vehicles by which the synergistic compositions can be formulated for delivery and use as a fungicide.

As will be readily appreciated, any material to which these synergistic compositions can be added may be used, provided they yield the desired utility without significant interference with the activity of these synergistic compositions as antifungal agents.

Wettable powders, which may be compacted to form water-dispersible granules, comprise an intimate mixture of the synergistic composition, a carrier and agriculturally acceptable surfactants. The concentration of the synergistic composition in the wettable powder is usually from about 10% to about 90% by weight, more preferably about 25% to about 75% by weight, based on the total weight of the formulation. In the preparation of wettable powder formulations, the synergistic composition can be compounded with any of the finely divided solids, such as prophyllite, talc, chalk, gypsum, Fuller's earth, bentonite, attapulgite, starch, casein, gluten, montmorillonite clays, diatomaceous earths, purified silicates or the like. In such operations, the finely divided carrier is ground or mixed with the synergistic composition in a volatile organic solvent. Effective surfactants, comprising from about 0.5% to about 10% by weight of the wettable powder, include sulfonated lignins, naphthalenesulfonates, alkylbenzenesulfonates, alkyl sulfates, and non-ionic surfactants, such as ethylene oxide adducts of alkyl phenols.

Emulsifiable concentrates of the synergistic composition comprise a convenient concentration, such as from about 10% to about 50% by weight, in a suitable liquid, based on the total weight of the emulsifiable concentrate formulation. The components of the synergistic compositions, jointly or separately, are dissolved in a carrier, which is either a water-miscible solvent or a mixture of water-immiscible organic solvents, and emulsifiers. The concentrates may be diluted with water and oil to form spray mixtures in the form of oil-in-water emulsions. Useful organic solvents include

aromatics, especially the high-boiling naphthalenic and olefinic portions of petroleum such as heavy aromatic naphtha. Other organic solvents may also be used, such as, for example, terpenic solvents, including rosin derivatives, aliphatic ketones, such as cyclohexanone, and complex alcohols, such as 2-ethoxyethanol.

Emulsifiers which can be advantageously employed herein can be readily determined by those skilled in the art and include various nonionic, anionic, cationic and amphoteric emulsifiers, or a blend of two or more emulsifiers. Examples of nonionic emulsifiers useful in preparing the emulsifiable concentrates include the polyalkylene glycol ethers and condensation products of alkyl and aryl phenols, aliphatic alcohols, aliphatic amines or fatty acids with ethylene oxide, propylene oxides such as the ethoxylated alkyl phenols and carboxylic esters solubilized with the polyol or polyoxyalkylene. Cationic emulsifiers include quaternary ammonium compounds and fatty amine salts. Anionic emulsifiers include the oil-soluble salts (e.g., calcium) of alkylaryl sulfonic acids, oil-soluble salts or sulfated polyglycol ethers and appropriate salts of phosphated polyglycol ether.

Representative organic liquids which can be employed in preparing the emulsifiable concentrates of the present disclosure are the aromatic liquids such as xylene, propyl benzene fractions, or mixed naphthalene fractions, mineral oils, substituted aromatic organic liquids such as dioctyl phthalate, kerosene, dialkyl amides of various fatty acids, particularly the dimethyl amides of fatty glycols and glycol derivatives such as the *n*-butyl ether, ethyl ether or methyl ether of diethylene glycol, and the methyl ether of triethylene glycol. Mixtures of two or more organic liquids are also often suitably employed in the preparation of the emulsifiable concentrate. The preferred organic liquids are xylene, and propyl benzene fractions, with xylene being most preferred. The surface-active dispersing agents are usually employed in liquid formulations and in the amount of from 0.1 to 20 percent by weight of the combined weight of the dispersing agent with the synergistic compositions. The formulations can also contain other compatible additives, for example, plant growth regulators and other biologically active compounds used in agriculture.

Aqueous suspensions comprise suspensions of one or more water-insoluble compounds, dispersed in an aqueous vehicle at a concentration in the range from about 5% to about 70% by weight, based on the total weight of the aqueous suspension formulation. Suspensions are prepared by finely grinding the components of the synergistic combination either together or separately, and vigorously mixing the ground material into a vehicle comprised of water and surfactants chosen from the same types discussed above. Other ingredients, such as inorganic salts and synthetic or natural gums, may also be added to increase the density and viscosity of the aqueous vehicle. It is often most effective to grind and mix at the same time by preparing the aqueous mixture and homogenizing it in an implement such as a sand mill, ball mill, or piston-type homogenizer.

The synergistic composition may also be applied as a granular formulation, which is particularly useful for applications to the soil. Granular formulations usually contain from about 0.5% to about 10% by weight of the compounds, based on the total weight of the granular formulation, dispersed in a carrier which consists entirely or in large part of coarsely divided attapulgite, bentonite, diatomite, clay or a similar inexpensive substance. Such formulations are usually prepared by dissolving the synergistic composition in a suitable solvent and applying it to a granular carrier which has been preformed to the appropriate particle size, in the range of from about 0.5 to about 3 millimeters (mm). Such formulations may also be prepared by making a dough or paste of the carrier and the synergistic composition, and crushing and drying to obtain the desired granular particle.

Dusts containing the synergistic composition are prepared simply by intimately mixing the synergistic composition in powdered form with a suitable dusty agricultural carrier, such as, for example, kaolin clay, ground volcanic rock, and the like. Dusts can suitably contain from about 1% to about 10% by weight of the synergistic composition/carrier combination.

The formulations may contain agriculturally acceptable adjuvant surfactants to enhance deposition, wetting and penetration of the synergistic composition onto the target crop and organism. These adjuvant surfactants may optionally be employed as a component of the formulation or as a tank mix. The amount of adjuvant surfactant will vary from 0.01 percent to 1.0 percent volume/volume (v/v) based on a spray-volume of water, preferably 0.05 to 0.5 percent. Suitable adjuvant surfactants include ethoxylated nonyl phenols, ethoxylated synthetic or natural alcohols, salts of the esters or sulfosuccinic acids, ethoxylated organosilicones, ethoxylated fatty amines and blends of surfactants with mineral or vegetable oils.

The formulations may optionally include combinations that can comprise at least 1% by weight of one or more of the synergistic compositions with another pesticidal compound. Such additional pesticidal compounds may be fungicides, insecticides, nematicides, miticides, arthropodicides, bactericides or combinations thereof that are compatible with the synergistic compositions of the present disclosure in the medium selected for application, and not antagonistic to the activity of the present compounds. Accordingly, in such embodiments the other pesticidal compound is employed as a supplemental toxicant for the same or for a different pesticidal use. The pesticidal compound and the synergistic composition can generally be mixed together in a weight ratio of from 1:100 to 100:1.

The present disclosure includes within its scope methods for the control or prevention of fungal attack. These methods comprise applying to the locus of the fungus, or to a locus in which the infestation is to be prevented (for example applying to tomato plants), a fungicidally effective amount of the synergistic composition. The synergistic

composition is suitable for treatment of various plants at fungicidal levels, while exhibiting low phytotoxicity. The synergistic composition is useful in a protectant or eradicant fashion. The synergistic composition is applied by any of a variety of known techniques, either as the synergistic composition or as a formulation comprising the synergistic composition. For example, the synergistic compositions may be applied to the roots, seeds or foliage of plants for the control of various fungi, without damaging the commercial value of the plants. The synergistic composition is applied in the form of any of the generally used formulation types, for example, as solutions, dusts, wettable powders, flowable concentrates, or emulsifiable concentrates. These materials are conveniently applied in various known fashions.

The synergistic composition has been found to have significant fungicidal effect, particularly for agricultural use. The synergistic composition is particularly effective for use with agricultural crops and horticultural plants, or with wood, paint, leather or carpet backing.

In particular, the synergistic composition is effective in controlling a variety of undesirable fungi that infect useful plant crops. The synergistic composition may be used against a variety of *Ascomycete* and *Basidiomycete* fungi, including for example the following representative fungi species: barley leaf scald (*Rhynchosporium secalis*); barley Ramularia leaf spot (*Ramularia collo-cygni*); barley net blotch (*Pyrenophora teres*); barley powdery mildew (*Blumeria graminis* f. sp. *hordei*); wheat powdery midlew (*Blumeria graminis* f. sp. *tritici*); wheat brown rust (*Puccinia triticina*); stripe rust of wheat (*Puccinia striiformis*); leaf blotch of wheat (*Zymoseptoria tritici*); glume blotch of wheat (*Parastagonospora nodorum*); leaf spot of sugar beets (*Cercospora beticola*); leaf spot of peanut (*Mycosphaerella arachidis*); cucumber anthracnose (*Colletotrichum lagenarium*); cucumber powdery mildew (*Podosphaera xanthii*); watermelon stem gummy blight (*Didymella bryoniae*); apple scab (*Venturia inaequalis*); apple powdery mildew (*Podosphaera leucotricha*); grey mold (*Botrytis cinerea*); Sclerotinia white mold (*Sclerotinia sclerotiorum*); grape powdery mildew (*Erysiphe necator*); rice blast (*Pyricularia oryzae*); brown rot of stone fruits (*Monilinia fructicola*) and black sigatoka disease of banana (*Mycosphaerella fijiensis*). It will be understood by those in the art that the efficacy of the synergistic compositions for one or more of the foregoing fungi establishes the general utility of the synergistic compositions as fungicides.

The synergistic compositions have a broad range of efficacy as a fungicide. The exact amount of the synergistic composition to be applied is dependent not only on the relative amounts of the components, but also on the particular action desired, the fungal species to be controlled, and the stage of growth thereof, as well as the part of the plant or other product to be contacted with the synergistic composition. Thus, formulations containing the synergistic composition may not be equally effective at similar concentrations or against the same fungal species.

The synergistic compositions are effective in use with plants in a disease-inhibiting and phytologically acceptable amount. The term "disease-inhibiting and phytologically acceptable amount" refers to an amount of the synergistic composition that kills or inhibits the plant disease for which control is desired, but is not significantly toxic to the plant. The exact concentration of synergistic composition required varies with the fungal disease to be controlled, the type of formulation employed, the method of application, the particular plant species, climate conditions, and the like.

The present compositions can be applied to fungi or their locus by the use of conventional ground sprayers, granule applicators, and by other conventional means known to those skilled in the art.

Some aspects of the present invention are summarized in the following numbered clauses:

1. A synergistic fungicidal mixture, comprising:

a fungicidally effective amount of the compound of Formula I, (*S*)-1,1-bis(4-fluorophenyl)propan-2-yl (3-acetoxy-4-methoxypicolinoyl)-L-alaninate:

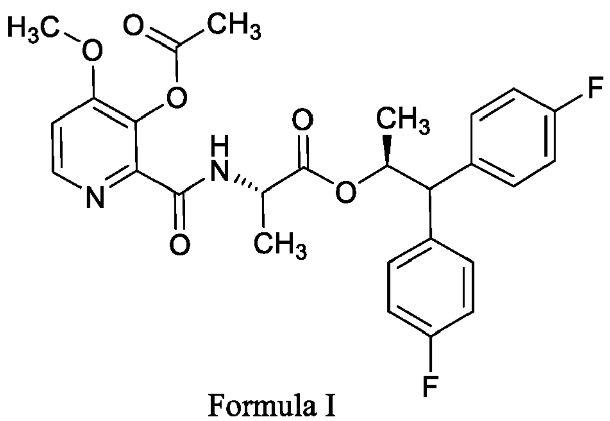

Formula I

;

and

at least one additional fungicide selected from the group consisting of sterol biosynthesis inhibitors, respiration inhibitors, and multi-site action inhibitors.

2. The mixture of clause 1 wherein the sterol biosynthesis inhibitor is selected from the group consisting of epoxiconazole, prothioconazole and difenoconazole.
3. The mixture of clause 2 wherein a concentration ratio of the compound of Formula I to epoxiconazole is from about 10:1 to about 1:1.6.
4. The mixture of clause 2 wherein a concentration ratio of the compound of Formula I to prothioconazole is from about 10:1 to about 1.3:1.
5. The mixture of clause 2 wherein a concentration ratio of the compound of Formula I to difenoconazole is from about 5:1 to about 1:1.6.
6. The mixture of clause 1 wherein the respiration inhibitor is selected from the group consisting of azoxystrobin, pyraclostrobin and picoxystrobin.
7. The mixture of clause 6 wherein a concentration ratio of the compound of Formula I to azoxystrobin is from about 50:1 to about 3.1:1.
8. The mixture of clause 6 wherein a concentration ratio of the compound of Formula I to pyraclostrobin is from about 50:1 to about 3.1:1.
9. The mixture of clause 6 wherein a concentration ratio of the compound of Formula I to picoxystrobin is from about 10:1 to about 1:1.6.
10. The mixture of clause 1 wherein the respiration inhibitor is selected from the group consisting of fluxapyroxad, benzovindiflupyr, penthiopyrad and bixafen.
11. The mixture of clause 10 wherein a concentration ratio of the compound of Formula I to fluxapyroxad is from about 50:1 to about 3.1:1.
12. The mixture of clause 10 wherein a concentration ratio of the compound of Formula I to benzovindiflupyr is from about 10:1 to about 1:1.6.
13. The mixture of clause 10 wherein a concentration ratio of the compound of Formula I to penthiopyrad is from about 10:1 to about 1.3:1.
14. The mixture of clause 10 wherein a concentration ratio of the compound of Formula I to bixafen is from about 10:1 to about 1:1.6.
15. The mixture of clause 1 wherin the multi-site inhibitor is selected from the group consisting of chlorothalonil and mancozeb.
16. The mixture of clause 15 wherein a concentration ratio of the compound of Formula I to chlorothalonil is from about 1:1.3 to about 1:20.
17. The mixture of clause 15 wherein a concentration ratio of the compound of Formula I to mancozeb is from about 1:1.3 to about 1:20.
18. The mixture of clauses 1-17, wherein the mixture provides control of a fungal pathogen, and the pathogen is the causal agent of early blight of tomato (*Alternaria solani*).
19. A synergistic, fungicidal composition comprising of a fungicidally effective amount of the mixtures of clauses 1 - 17 and an agriculturally acceptable carrier.

The following examples are provided for illustrative purposes and should not be construed as limitations to the disclosure.

Examples

Evaluation of Protectant Activity of Fungicide Mixtures vs. Tomato Early Blight (*Alternaria solani*; Bayer code: ALTESO):
Tomato seedlings (variety Outdoor Girl) were propagated in a soil-less media (Metro mix™), with each pot having 1 plant, and used in the test when the first set of leaves was fully expanded. Treatments consisted of fungicide compounds epoxiconazole, prothioconazole, difenoconazole, azoxystrobin, pyraclostrobin, picoxystrobin, fluxapyroxad, benzovindiflupyr, penthiopyrad, bixafen, chlorothalonil, and mancozeb either using individually or as two-way mixture with the compound of Formula I.
The compounds were tested as technical grade material formulated in acetone, and spray solutions contained 10% acetone and 100 ppm Triton X-100. Fungicide solutions were applied onto plants using an automated booth sprayer, which utilized two 6218-1/4 JAUPM spray nozzles operating at 20 pounds per square inch (psi) set at opposing angles to cover both leaf surfaces. All sprayed plants were allowed to air dry prior to further handling. Control plants were sprayed in the same manner with the solvent blank.
Test plants were inoculated with an aqueous spore suspension of *Alternaria solani* 1 day after fungicide treatments (1-day protectant test). After inoculation the plants were kept in 100% relative humidity for two days to permit spores to germinate and infect the leaf. The plants were then transferred to a growth chamber for disease to develop. When the disease was fully developed on untreated plants, disease severity on the seedlings was assessed and activity was represented by percent of leaf area free of ALTESO infection relative to the untreated plants. The percent disease control was calculated using the equation (1-(disease severity on treated plants/disease severity on untreated plants))* 100.

Colby's equation was used to determine the fungicidal effects expected from the mixtures. (See Colby, S. R. Calculation of the synergistic and antagonistic response of herbicide combinations. Weeds 1967, 15, 20-22.)

The following equation was used to calculate the expected activity of mixtures containing two active ingredients, A and B:

$$\text{Expected} = A + B - (A \times B/100)$$

A = observed efficacy of active component A at the same concentration as used in the mixture;
B = observed efficacy of active component B at the same concentration as used in the mixture.

Synergistic interactions between compound I and other fungicides were detected in protectant assays vs. ALTESO (Table 1).

Table 1: Synergistic Interactions of the Compound of Formula I and Other Fungicides in a 1-Day Protectant (1DP) *Alternaria solani* (ALTESO) Assay.

| Composition | Rates (ppm)* | ALTESO* | | Synergism Factor* |
|---|---|---|---|---|
| | | Observed* | Expected* | |
| Epoxiconazole + Compound I | 4 + 10 | 93.3 | 16.3 | 5.7 |
| Epoxiconazole + Compound I | 2 + 10 | 65.0 | 12.7 | 5.1 |
| Epoxiconazole + Compound I | 1 + 10 | 61.7 | 10.0 | 6.2 |
| Epoxiconazole + Compound I | 4+5 | 75.0 | 22.8 | 3.3 |
| Epoxiconazole + Compound I | 2+5 | 71.7 | 19.5 | 3.7 |
| Epoxiconazole + Compound I | 1 + 5 | 63.3 | 17.0 | 3.7 |
| Epoxiconazole + Compound I | 4 + 2.5 | 16.7 | 9.8 | 1.7 |
| Epoxiconazole + Compound I | 2 + 2.5 | 23.3 | 5.9 | 3.9 |
| Epoxiconazole + Compound I | 1 + 2.5 | 13.3 | 3.0 | 4.4 |
| Prothioconazole + Compound I | 2 + 10 | 33.3 | 10.0 | 3.3 |
| Prothioconazole + Compound I | 1 + 10 | 26.7 | 19.0 | 1.4 |
| Prothioconazole + Compound I | 4+5 | 60.0 | 41.9 | 1.4 |
| Prothioconazole + Compound I | 2+5 | 43.3 | 17.0 | 2.5 |

(continued)

| Composition | Rates (ppm)* | ALTESO* | | Synergism Factor* |
|---|---|---|---|---|
| | | Observed* | Expected* | |
| Prothioconazole + Compound I | 1 + 5 | 56.7 | 25.3 | 2.2 |
| Prothioconazole + Compound I | 2 + 2.5 | 36.7 | 3.0 | 12.2 |
| Prothioconazole + Compound I | 1 + 2.5 | 46.7 | 12.7 | 3.7 |
| Difenoconazole + Compound I | 4 + 10 | 79.0 | 31.0 | 2.5 |
| Difenoconazole + Compound I | 2 + 10 | 50.0 | 34.0 | 1.5 |
| Difenoconazole + Compound I | 4+5 | 90.7 | 36.4 | 2.5 |
| Difenoconazole + Compound I | 2+5 | 63.3 | 39.1 | 1.6 |
| Difenoconazole + Compound I | 4 + 2.5 | 53.3 | 25.6 | 2.1 |
| Difenoconazole + Compound I | 2 + 2.5 | 43.3 | 28.9 | 1.5 |
| Azoxystrobin + Compound I | 0.8 + 10 | 80.0 | 64.0 | 1.3 |
| Azoxystrobin + Compound I | 0.4 + 10 | 83.3 | 31.0 | 2.7 |
| Azoxystrobin + Compound I | 0.2 + 10 | 61.7 | 22.0 | 2.8 |
| Azoxystrobin + Compound I | 0.8 + 5 | 80.0 | 66.8 | 1.2 |
| Azoxystrobin + Compound I | 0.4 + 5 | 83.3 | 36.4 | 2.3 |
| Azoxystrobin + Compound I | 0.2 + 5 | 63.3 | 28.1 | 2.3 |
| Azoxystrobin + Compound I | 0.8 + 2.5 | 80.0 | 61.2 | 1.3 |
| Azoxystrobin + Compound I | 0.4 + 2.5 | 80.0 | 25.6 | 3.1 |
| Azoxystrobin + Compound I | 0.2 + 2.5 | 66.7 | 15.9 | 4.2 |
| Pyraclostrobin + Compound I | 0.8 + 10 | 78.3 | 31.0 | 2.5 |
| Pyraclostrobin + Compound I | 0.4 + 10 | 70.0 | 26.5 | 2.6 |
| Pyraclostrobin + Compound I | 0.2 + 10 | 66.7 | 13.0 | 5.1 |

(continued)

| Composition | Rates (ppm)* | ALTESO* | | Synergism Factor* |
|---|---|---|---|---|
| | | Observed* | Expected* | |
| Pyraclostrobin + Compound I | 0.8 + 5 | 53.3 | 36.4 | 1.5 |
| Pyraclostrobin + Compound I | 0.4 + 5 | 60.0 | 32.2 | 1.9 |
| Pyraclostrobin + Compound I | 0.2 + 5 | 66.7 | 19.8 | 3.4 |
| Pyraclostrobin + Compound I | 0.8 + 2.5 | 86.7 | 25.6 | 3.4 |
| Pyraclostrobin + Compound I | 0.4 + 2.5 | 65.0 | 20.8 | 3.1 |
| Pyraclostrobin + Compound I | 0.2 + 2.5 | 43.3 | 6.2 | 7.0 |
| Picoxystrobin + Compound I | 4 + 10 | 97.3 | 55.0 | 1.8 |
| Picoxystrobin + Compound I | 2 + 10 | 91.7 | 52.0 | 1.8 |
| Picoxystrobin + Compound I | 1 + 10 | 91.7 | 22.0 | 4.2 |
| Picoxystrobin + Compound I | 4+5 | 72.3 | 58.5 | 1.2 |
| Picoxystrobin + Compound I | 2+5 | 96.3 | 55.7 | 1.7 |
| Picoxystrobin + Compound I | 1 + 5 | 65.0 | 28.1 | 2.3 |
| Picoxystrobin + Compound I | 4 + 2.5 | 96.3 | 51.5 | 1.9 |
| Picoxystrobin + Compound I | 2 + 2.5 | 98.0 | 48.3 | 2.0 |
| Picoxystrobin + Compound I | 1 + 2.5 | 93.3 | 15.9 | 5.9 |
| Fluxapyroxad + Compound I | 0.8 + 10 | 46.7 | 19.0 | 2.5 |
| Fluxapyroxad + Compound I | 0.4 + 10 | 43.3 | 19.0 | 2.3 |
| Fluxapyroxad + Compound I | 0.2 + 10 | 53.3 | 31.0 | 1.7 |
| Fluxapyroxad + Compound I | 0.8 + 5 | 30.0 | 25.3 | 1.2 |
| Fluxapyroxad + Compound I | 0.8 + 2.5 | 33.3 | 12.7 | 2.6 |
| Fluxapyroxad + Compound I | 0.4 + 2.5 | 30.0 | 12.7 | 2.4 |

(continued)

| Composition | Rates (ppm)* | ALTESO* | | Synergism Factor* |
|---|---|---|---|---|
| | | Observed* | Expected* | |
| Fluxapyroxad + Compound I | 0.2 + 2.5 | 33.3 | 25.6 | 1.3 |
| Benzovindiflupyr + Compound I | 4 + 10 | 58.3 | 23.5 | 2.5 |
| Benzovindiflupyr + Compound I | 2 + 10 | 53.3 | 13.0 | 4.1 |
| Benzovindiflupyr + Compound I | 1 + 10 | 30.0 | 16.0 | 1.9 |
| Benzovindiflupyr + Compound I | 4+5 | 50.0 | 29.5 | 1.7 |
| Benzovindiflupyr + Compound I | 2+5 | 56.7 | 19.8 | 2.9 |
| Benzovindiflupyr + Compound I | 1 + 5 | 53.3 | 22.5 | 2.4 |
| Benzovindiflupyr + Compound I | 4 + 2.5 | 46.7 | 17.6 | 2.7 |
| Benzovindiflupyr + Compound I | 2 + 2.5 | 38.3 | 6.2 | 6.1 |
| Benzovindiflupyr + Compound I | 1 + 2.5 | 26.7 | 9.5 | 2.8 |
| Penthiopyrad + Compound I | 2 + 10 | 28.3 | 19.0 | 1.5 |
| Penthiopyrad + Compound I | 1 + 10 | 25.0 | 13.0 | 1.9 |
| Penthiopyrad + Compound I | 4+5 | 61.7 | 41.9 | 1.5 |
| Penthiopyrad + Compound I | 2+5 | 33.3 | 25.3 | 1.3 |
| Penthiopyrad + Compound I | 1 + 5 | 33.3 | 19.8 | 1.7 |
| Bixafen + Compound I | 4 + 10 | 81.7 | 61.0 | 1.3 |
| Bixafen + Compound I | 2 + 10 | 86.7 | 46.0 | 1.9 |
| Bixafen + Compound I | 1 + 10 | 73.3 | 49.0 | 1.5 |
| Bixafen + Compound I | 4+5 | 92.3 | 64.0 | 1.4 |
| Bixafen + Compound I | 2+5 | 78.3 | 50.2 | 1.6 |
| Bixafen + Compound I | 1 + 5 | 65.0 | 53.0 | 1.2 |
| Bixafen + Compound I | 4 + 2.5 | 96.7 | 58.0 | 1.7 |
| Bixafen + Compound I | 2 + 2.5 | 93.3 | 41.8 | 2.2 |
| Bixafen + Compound I | 1 + 2.5 | 94.0 | 45.0 | 2.1 |
| Chlorothalonil + Compound I | 50 + 10 | 56.7 | 25.0 | 2.3 |

(continued)

| Composition | Rates (ppm)* | ALTESO* | | Synergism Factor* |
|---|---|---|---|---|
| | | Observed* | Expected* | |
| Chlorothalonil + Compound I | 25 + 10 | 53.3 | 22.0 | 2.4 |
| Chlorothalonil + Compound I | 12.5 + 10 | 55.0 | 13.0 | 4.2 |
| Chlorothalonil + Compound I | 50 + 2.5 | 26.7 | 19.2 | 1.4 |
| Chlorothalonil + Compound I | 12.5 + 2.5 | 13.3 | 6.2 | 2.1 |
| Mancozeb + Compound I | 50 + 10 | 66.7 | 17.5 | 3.8 |
| Mancozeb + Compound I | 25 + 10 | 56.7 | 11.5 | 4.9 |
| Mancozeb + Compound I | 12.5 + 10 | 55.0 | 10.0 | 5.5 |
| Mancozeb + Compound I | 50 + 5 | 58.3 | 23.9 | 2.4 |
| Mancozeb + Compound I | 25 + 5 | 51.7 | 18.4 | 2.8 |
| Mancozeb + Compound I | 12.5 + 5 | 51.7 | 17.0 | 3.0 |
| Mancozeb + Compound I | 50 + 2.5 | 51.7 | 11.1 | 4.7 |
| Mancozeb + Compound I | 25 + 2.5 | 38.3 | 4.6 | 8.3 |
| Mancozeb + Compound I | 12.5 + 2.5 | 33.3 | 3.0 | 11.1 |

*ALTESO = Tomato early blight; *Alternaria solani*
*Observed = Observed percent disease control at the test rates
*Expected = Percent disease control expected as predicted by the Colby equation
*ppm = Parts per million
*Synergism factor = Observed / Expected

## Claims

**1.** A synergistic fungicidal mixture, comprising:

a fungicidally effective amount of the compound of Formula I, (*S*)-1,1-bis(4-fluorophenyl)propan-2-yl (3-acetoxy-4-methoxypicolinoyl)-L-alaninate:

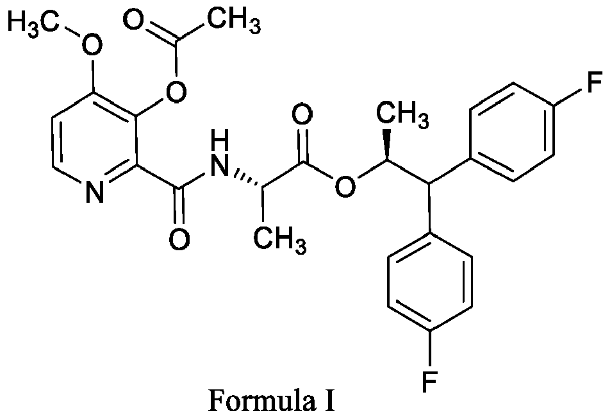

Formula I ;

and
at least one additional fungicide.

2. The mixture of claim 1 wherein selected from the group consisting of epoxiconazole, prothioconazole, difenoconazole, fluxapyroxad, benzovindiflupyr, penthiopyrad, bixafen, chlorothalonil and mancozeb.

3. The mixture of Claim 1 or 2, wherein the mixture provides control of a fungal pathogen, and the pathogen is the causal agent of early blight of tomato (*Alternaria solani*).

4. The mixture of any of Claims 1 - 3, wherein the concentration ratio of the compound of Formula I to other fungicide lies within the range from about 50:1 to about 1:20.

5. A synergistic, fungicidal composition comprising of a fungicidally effective amount of the mixtures of Claims 1 - 4 and an agriculturally acceptable carrier.

6. A method for controlling or preventing fungal attack in a plant comprising applying to the locus of the fungus or to a locus in which the infestation is to be prevented, a fungicidally effective amount of the synergistic mixture of claim 5.

7. The method of Claim 6, wherein the composition is applied to the roots, seed or foliage of the plants.

8. The method of Claim 6 or 7, wherein the composition is applied at a rate of between about 40 grams per hectare (g/ha) and about 2650 g/ha, based on the total amount of active ingredients in the composition.

Europäisches Patentamt
European Patent Office
Office européen des brevets

# EUROPEAN SEARCH REPORT

Application Number
EP 22 15 7915

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2016/109257 A1 (DOW AGROSCIENCES LLC [US]) 7 July 2016 (2016-07-07)<br>* paragraph [0039]; compound 145 *<br>----- | 1-8 | INV.<br>A01N43/54<br>A01N43/653<br>C07C229/08<br>A01P3/00<br>A01N43/40 |
| A | US 2011/082160 A1 (OWEN W JOHN [US] ET AL) 7 April 2011 (2011-04-07)<br>* tables 1-12 *<br>----- | 1-8 | |
| A | US 2014/187587 A1 (OUIMETTE DAVID G [US] ET AL) 3 July 2014 (2014-07-03)<br>* table 1 *<br>----- | 1-8 | |
| A | ANONYMOUS ED - DARL KUHN: "Synergistic Fungicidal Compositions of Heterocyclic Aromatic Amides and Triazoles",<br>IP.COM, IP.COM INC., WEST HENRIETTA, NY, US,<br>20 July 2004 (2004-07-20), XP013020905,<br>ISSN: 1533-0001<br>* tables 1-5 *<br>----- | 1-8 | |

| TECHNICAL FIELDS SEARCHED (IPC) |
|---|
| A01N |

The present search report has been drawn up for all claims

1

| Place of search | Date of completion of the search | Examiner |
|---|---|---|
| Munich | 11 August 2022 | Lorusso, Patrizia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 15 7915

This annex lists the patent family members relating to the patent documents cited in the above-mentioned European search report. The members are as contained in the European Patent Office EDP file on
The European Patent Office is in no way liable for these particulars which are merely given for the purpose of information.

11-08-2022

| Patent document cited in search report | Publication date | Patent family member(s) | Publication date |
|---|---|---|---|
| WO 2016109257 A1 | 07-07-2016 | AU 2015374427 A1 | 29-06-2017 |
| | | AU 2015380298 A1 | 29-06-2017 |
| | | AU 2018206798 A1 | 09-08-2018 |
| | | AU 2019201931 A1 | 11-04-2019 |
| | | AU 2021200664 A1 | 04-03-2021 |
| | | BR 112017013641 A2 | 13-03-2018 |
| | | BR 112017013676 A2 | 09-01-2018 |
| | | BR 122019023756 B1 | 25-01-2022 |
| | | BR 122019026066 B1 | 18-01-2022 |
| | | CA 2972401 A1 | 04-08-2016 |
| | | CA 2972405 A1 | 07-07-2016 |
| | | CL 2017001710 A1 | 26-01-2018 |
| | | CL 2017001711 A1 | 26-01-2018 |
| | | CN 107205405 A | 26-09-2017 |
| | | CN 107207414 A | 26-09-2017 |
| | | CN 113173838 A | 27-07-2021 |
| | | CN 113615696 A | 09-11-2021 |
| | | CO 2017006845 A2 | 30-11-2017 |
| | | CO 2017006857 A2 | 30-11-2017 |
| | | CR 20170307 A | 10-08-2017 |
| | | CR 20170308 A | 10-08-2017 |
| | | CR 20220034 A | 11-02-2022 |
| | | DK 3240773 T3 | 07-09-2020 |
| | | EC SP17044702 A | 30-11-2017 |
| | | EC SP17044745 A | 30-11-2017 |
| | | EP 3240424 A1 | 08-11-2017 |
| | | EP 3240773 A1 | 08-11-2017 |
| | | EP 3808181 A1 | 21-04-2021 |
| | | ES 2815673 T3 | 30-03-2021 |
| | | ES 2838774 T3 | 02-07-2021 |
| | | GT 201700147 A | 23-11-2018 |
| | | GT 201700148 A | 23-11-2018 |
| | | HK 1245019 A1 | 24-08-2018 |
| | | HK 1245236 A1 | 24-08-2018 |
| | | IL 273591 A | 31-05-2020 |
| | | JP 6684810 B2 | 22-04-2020 |
| | | JP 6687625 B2 | 22-04-2020 |
| | | JP 2018502103 A | 25-01-2018 |
| | | JP 2018505864 A | 01-03-2018 |
| | | JP 2020121983 A | 13-08-2020 |
| | | JP 2022003033 A | 11-01-2022 |
| | | KR 20170102259 A | 08-09-2017 |
| | | KR 20170102260 A | 08-09-2017 |
| | | NZ 732641 A | 21-12-2018 |
| | | NZ 732820 A | 21-12-2018 |
| | | PH 12017501196 A1 | 18-10-2017 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 22 15 7915

This annex lists the patent family members relating to the patent documents cited in the above-mentioned European search report. The members are as contained in the European Patent Office EDP file on
The European Patent Office is in no way liable for these particulars which are merely given for the purpose of information.

11-08-2022

| Patent document cited in search report | Publication date | Patent family member(s) | Publication date |
|---|---|---|---|
| | | PH 12017501197 A1 | 18-10-2017 |
| | | PL 3240773 T3 | 16-11-2020 |
| | | PT 3240773 T | 10-09-2020 |
| | | RU 2017123622 A | 31-01-2019 |
| | | RU 2017123819 A | 31-01-2019 |
| | | RU 2019138183 A | 20-12-2019 |
| | | TW 201627274 A | 01-08-2016 |
| | | TW 201627287 A | 01-08-2016 |
| | | US 2017290333 A1 | 12-10-2017 |
| | | US 2017295792 A1 | 19-10-2017 |
| | | US 2018228159 A1 | 16-08-2018 |
| | | US 2019059383 A1 | 28-02-2019 |
| | | US 2020120936 A1 | 23-04-2020 |
| | | US 2022159962 A1 | 26-05-2022 |
| | | UY 36480 A | 29-07-2016 |
| | | UY 36481 A | 29-07-2016 |
| | | WO 2016109257 A1 | 07-07-2016 |
| | | WO 2016122802 A1 | 04-08-2016 |
| | | ZA 201704554 B | 28-11-2018 |
| | | ZA 201704558 B | 28-11-2018 |
| US 2011082160 A1 | 07-04-2011 | AU 2010303529 A1 | 26-04-2012 |
| | | AU 2015200857 A1 | 12-03-2015 |
| | | BR 112012008010 A2 | 25-08-2020 |
| | | CA 2776746 A1 | 14-04-2011 |
| | | CL 2012000888 A1 | 31-08-2012 |
| | | CL 2015002092 A1 | 27-11-2015 |
| | | CN 102711477 A | 03-10-2012 |
| | | CN 105409971 A | 23-03-2016 |
| | | CO 6541551 A2 | 16-10-2012 |
| | | CR 20120195 A | 01-06-2012 |
| | | CR 20180237 A | 11-09-2018 |
| | | CR 20180239 A | 11-09-2018 |
| | | DK 2485592 T3 | 05-09-2016 |
| | | DK 2904901 T3 | 02-10-2017 |
| | | DK 3153020 T3 | 25-03-2019 |
| | | DK 3153021 T3 | 18-03-2019 |
| | | DK 3178321 T3 | 26-08-2019 |
| | | EC SP12011870 A | 31-07-2012 |
| | | EP 2485592 A1 | 15-08-2012 |
| | | EP 2904901 A2 | 12-08-2015 |
| | | EP 3153020 A1 | 12-04-2017 |
| | | EP 3153021 A1 | 12-04-2017 |
| | | EP 3178321 A1 | 14-06-2017 |
| | | ES 2580204 T3 | 22-08-2016 |
| | | ES 2639305 T3 | 26-10-2017 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO.

EP 22 15 7915

This annex lists the patent family members relating to the patent documents cited in the above-mentioned European search report. The members are as contained in the European Patent Office EDP file on
The European Patent Office is in no way liable for these particulars which are merely given for the purpose of information.

11-08-2022

| Patent document cited in search report | Publication date | Patent family member(s) | Publication date |
|---|---|---|---|
| | | ES 2712081 T3 | 09-05-2019 |
| | | ES 2712448 T3 | 13-05-2019 |
| | | ES 2735798 T3 | 20-12-2019 |
| | | HK 1174203 A1 | 07-06-2013 |
| | | HK 1222298 A1 | 30-06-2017 |
| | | HU E029892 T2 | 28-04-2017 |
| | | HU E034376 T2 | 28-02-2018 |
| | | HU E041611 T2 | 28-05-2019 |
| | | HU E042624 T2 | 29-07-2019 |
| | | HU E044673 T2 | 28-11-2019 |
| | | IL 219032 A | 28-09-2017 |
| | | JP 5905826 B2 | 20-04-2016 |
| | | JP 6134404 B2 | 24-05-2017 |
| | | JP 6352489 B2 | 04-07-2018 |
| | | JP 2013507372 A | 04-03-2013 |
| | | JP 2016128496 A | 14-07-2016 |
| | | JP 2017171669 A | 28-09-2017 |
| | | KR 20120093925 A | 23-08-2012 |
| | | KR 20170063986 A | 08-06-2017 |
| | | KR 20180069123 A | 22-06-2018 |
| | | LT 2904901 T | 11-09-2017 |
| | | LT 3153020 T | 11-02-2019 |
| | | LT 3153021 T | 25-02-2019 |
| | | LT 3178321 T | 10-06-2019 |
| | | MX 355100 B | 05-04-2018 |
| | | MX 355101 B | 05-04-2018 |
| | | MY 175147 A | 10-06-2020 |
| | | NZ 599319 A | 27-06-2014 |
| | | NZ 625074 A | 27-11-2015 |
| | | NZ 713736 A | 27-01-2017 |
| | | PH 12015501443 A1 | 14-09-2020 |
| | | PL 2485592 T3 | 31-08-2017 |
| | | PL 2904901 T3 | 31-01-2018 |
| | | PL 3153020 T3 | 31-05-2019 |
| | | PL 3153021 T3 | 31-05-2019 |
| | | PL 3178321 T3 | 31-10-2019 |
| | | PT 2485592 T | 27-07-2016 |
| | | PT 2904901 T | 02-10-2017 |
| | | PT 3153020 T | 26-02-2019 |
| | | PT 3153021 T | 19-03-2019 |
| | | PT 3178321 T | 01-08-2019 |
| | | RU 2012118656 A | 20-11-2013 |
| | | RU 2015106010 A | 20-07-2015 |
| | | TR 201900215 T4 | 21-02-2019 |
| | | TR 201900242 T4 | 21-02-2019 |
| | | TR 201908611 T4 | 22-07-2019 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 22 15 7915

This annex lists the patent family members relating to the patent documents cited in the above-mentioned European search report. The members are as contained in the European Patent Office EDP file on
The European Patent Office is in no way liable for these particulars which are merely given for the purpose of information.

11-08-2022

| Patent document cited in search report | Publication date | Patent family member(s) | Publication date |
|---|---|---|---|
| | | UA 106245 C2 | 11-08-2014 |
| | | UA 115314 C2 | 25-10-2017 |
| | | US 2011082160 A1 | 07-04-2011 |
| | | US 2015065529 A1 | 05-03-2015 |
| | | US 2018242583 A1 | 30-08-2018 |
| | | WO 2011044213 A1 | 14-04-2011 |
| | | ZA 201202424 B | 26-06-2013 |
| US 2014187587 A1 | 03-07-2014 | AR 094305 A1 | 22-07-2015 |
| | | AU 2013370491 A1 | 02-07-2015 |
| | | BR 112015015351 A2 | 11-07-2017 |
| | | CA 2894515 A1 | 03-07-2014 |
| | | CL 2015001841 A1 | 02-10-2015 |
| | | CN 104883884 A | 02-09-2015 |
| | | CR 20150304 A | 24-08-2015 |
| | | DK 2938191 T3 | 07-05-2018 |
| | | EC SP15032758 A | 30-11-2015 |
| | | EP 2938191 A1 | 04-11-2015 |
| | | ES 2666144 T3 | 03-05-2018 |
| | | HK 1215356 A1 | 26-08-2016 |
| | | HU E038806 T2 | 28-11-2018 |
| | | JP 6352302 B2 | 04-07-2018 |
| | | JP 2016505606 A | 25-02-2016 |
| | | KR 20150100865 A | 02-09-2015 |
| | | LT 2938191 T | 10-05-2018 |
| | | NZ 708979 A | 31-01-2020 |
| | | PH 12015501475 A1 | 01-02-2016 |
| | | PL 2938191 T3 | 31-07-2018 |
| | | PT 2938191 T | 09-05-2018 |
| | | RU 2015131103 A | 31-01-2017 |
| | | TW 201427597 A | 16-07-2014 |
| | | TW 201725987 A | 01-08-2017 |
| | | UA 114661 C2 | 10-07-2017 |
| | | US 2014187587 A1 | 03-07-2014 |
| | | UY 35247 A | 31-07-2014 |
| | | WO 2014105842 A1 | 03-07-2014 |
| | | ZA 201504334 B | 28-09-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62500186


### Non-patent literature cited in the description


- The Pesticide Manual. 2009
- The e-Pesticide Manual. 2011
- The Pesticide Manual. 2006
- **COLBY, S. R.** Calculation of the synergistic and antagonistic response of herbicide combinations. *Weeds,* 1967, vol. 15, 20-22